# FASCICULE DE BREVET EUROPEEN

(11) **EP 1 400 210 B1**
(45) Date de publication et mention de la délivrance du brevet: **12.11.2008**
(21) Numéro de dépôt: 03291718.9
(22) Date de dépôt: 10.07.2003
(51) Int. Cl.: A61B 17/22

(54) **Extracteur chirugical pour l'extration de corps étrangers à travers des voies naturelles ou chirurgicales**
Chirurgischer Extraktor zum Entfernen von Fremdkörpern über natürliche oder chirurgische Kanäle
Surgical extractor for removing foreign bodies across natural or surgical tracts

(30) Priorité: 17.09.2002 FR 0211501
(43) Date de publication de la demande: 24.03.2004
(73) Titulaire: Coloplast A/S, 3050 Humlebaek (DK)
(72) Inventeur: Torchio, Gérard, 91370 Verrieres le Buisson (FR)
(74) Mandataire: Thurgood, Alexander John

(56) Documents cités:
- US-A- 5 989 266
- US-B1- 6 224 612

## Description

La présente invention concerne un extracteur chirurgical pour l'extraction de corps étrangers, par exemple des calculs urinaires ou biliaires, à travers des voies naturelles ou chirurgicales.

Par exemple par le brevet américain 4 347 846, on connaît déjà un tel extracteur chirurgical comportant :
- une gaine souple apte à pénétrer dans lesdites voies jusqu'à un tel corps à extraire ;
- un fil de manoeuvre longitudinalement rigide, apte à coulisser dans ladite gaine souple et à être manoeuvré en coulissement, depuis l'extérieur, par son extrémité proximale ; et
- une pluralité de fils d'extraction disposés à l'extrémité distale dudit fil de manoeuvre et pouvant prendre, sous l'action de ce dernier :
   . soit une position de capture, pour laquelle ils sont arqués et écartés les uns des autres en formant, à l'extérieur de l'extrémité distale de ladite gaine souple, une cage ajourée en forme au moins approximative de globe, dont chaque fil arqué forme un méridien,
   . soit une position d'extraction, pour laquelle ils sont proches les uns des autres et rétractés au moins partiellement à l'intérieur de la partie distale de ladite gaine souple.

Dans un tel extracteur connu, tous lesdits fils d'extraction sont solidaires les uns des autres à leurs extrémités distales par soudage ou analogue, en formant une queue saillante à l'extrémité distale de ladite cage.

Une telle queue saillante présente l'inconvénient d'empêcher l'extrémité distale de l'extracteur de s'approcher au plus près de la paroi de l'organe contenant lesdits corps étrangers pour capturer ceux qui se trouvent au voisinage de ladite paroi.

Aussi, pour remédier à cet inconvénient, on a déjà prévu (voir par exemple WO 99/16364 et WO 99/53849) de supprimer ladite queue saillante en réalisant d'un seul tenant chaque couple de fils méridiens se trouvant dans un même plan méridien de ladite cage. Ainsi, cette dernière est alors formée d'une pluralité de boucles méridiennes indépendantes se croisant à l'extrémité distale de ladite cage.

Si une telle solution permet effectivement de supprimer la queue distale mentionnée ci-dessus, et donc de saisir des corps étrangers proches de la paroi de l'organe qui les contient, elle présente en revanche l'inconvénient que les boucles méridiennes indépendantes sont libres les unes par rapport aux autres de sorte que leurs positions relatives et leurs équerrages peuvent varier de façon importante, ce qui entraîne des difficultés pour saisir et/ou extraire lesdits corps étrangers.

Pour remédier à ce nouvel inconvénient, on a alors proposé (voir par exemple US-5 057 114, US-5 484 384 et US-5 989 266) de solidariser lesdites boucles méridiennes entre elles à leurs extrémités distales, là où elles se croisent. Cependant, ce faisant, on communique à ladite cage une certaine rigidité l'empêchant de s'adapter à un corps étranger à extraire et à l'environnement dans lequel se trouve celui-ci.

L'objet de la présente invention est de remédier aussi bien à ce dernier inconvénient qu'à celui résultant de l'indépendance desdites boucles méridiennes.

A cette fin, selon l'invention, l'extracteur chirurgical pour l'extraction de corps étrangers à travers des voies naturelles ou chirurgicales, comportant :
- une gaine souple apte à pénétrer dans lesdites voies jusqu'à un tel corps à extraire ;
- un fil de manoeuvre longitudinalement rigide, apte à coulisser dans ladite gaine souple et à être manoeuvré en coulissement, depuis l'extérieur, par son extrémité proximale ; et
- deux boucles de fil disposées à l'extrémité distale dudit fil de manoeuvre et pouvant prendre, sous l'action de ce dernier :
   - soit une position de capture, pour laquelle lesdites boucles sont déployées en formant, à l'extérieur de l'extrémité distale de ladite gaine souple, une cage ajourée en forme au moins approximative de globe, dont chaque boucle forme un plan méridien,
   - soit une position d'extraction, pour laquelle lesdites boucles sont aplaties et rétractées au moins partiellement à l'intérieur de la partie distale de ladite gaine souple,
   lesdites boucles de fil se croisant à leurs extrémités distales en y étant liées les unes aux autres,
est remarquable en ce que, à son extrémité distale, le fil d'une desdites boucles de fil comporte un passage dans lequel passe avec jeu l'autre boucle de fil, de sorte que, à l'emplacement du croisement distal lesdites boucles de fil sont orthogonales et celles-ci sont liées en coulissement, chaque boucle pouvant coulisser de façon limitée par rapport à au moins une autre boucle tout en gardant au moins approximativement, en position de capture, son équerrage par rapport à ladite autre boucle.

Aussi, un extracteur chirurgical selon la revendication 2 peut remédier aussi bien à ce dernier inconvénient.

Ainsi, on communique aux boucles dudit globe une indépendance limitée leur permettant de s'adapter au mieux aux corps étrangers à extraire et à leur environnement, tout en ne dégradant pas les propriétés d'extraction dudit extracteur.

Un tel passage peut être formé entre le fil de ladite boucle correspondante et une languette découpée dans ce dernier fil et conformée de façon adéquate.

Les figures du dessin annexé feront bien comprendre comment l'invention peut être réalisée. Sur ces figures, des références identiques désignent des éléments semblables.

La figure 1 est une vue, partielle et schématique, de la partie distale d'un exemple de réalisation de l'extracteur chirurgical conforme à la présente invention, en position de capture déployée.

Les figures 2 et 3 illustrent, à plus grande échelle et en vue selon la flèche F de la figure 1, deux variantes de réalisation du croisement coulissant des extrémités distales des boucles de fil dudit exemple de réalisation.

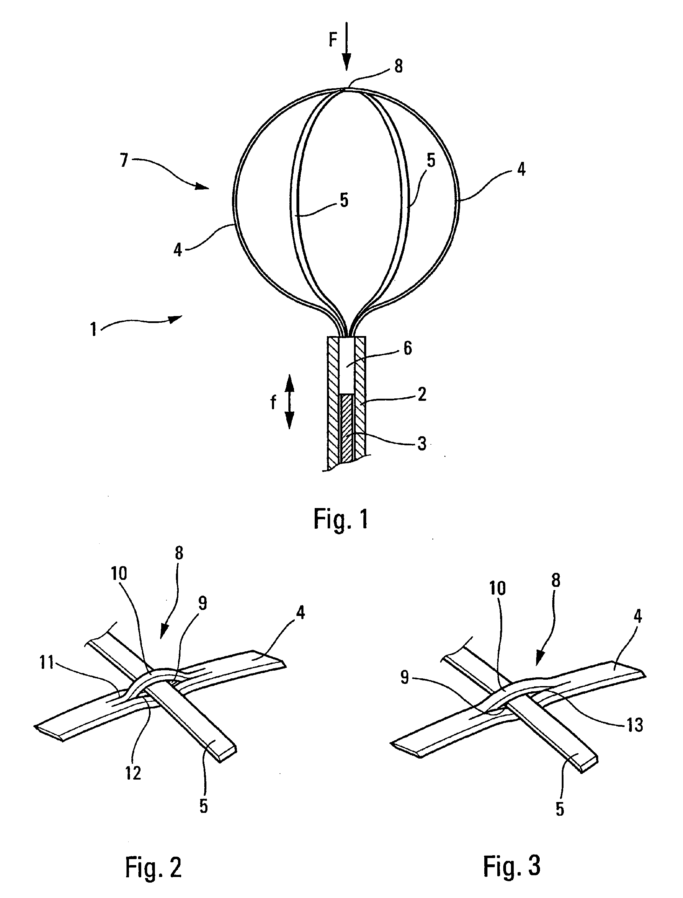

L'extracteur chirurgical 1 pour l'extraction de corps étrangers à travers des voies naturelles ou chirurgicales (non représentées), conforme à la présente invention et représenté sur la figure 1, comporte :
- une gaine souple 2, apte à pénétrer dans lesdites voies jusqu'à un corps à extraire (non représenté) ;
- un fil de manoeuvre 3 longitudinalement rigide, par exemple constitué par un câble apte à coulisser dans ladite gaine souple 2 (comme illustré par la flèche f) et à être manoeuvré en coulissement, depuis l'extérieur, par son extrémité proximale (non représentée) ; et
- deux boucles de fil 4 et 5, par exemple en alliage à mémoire de forme tel que le Nitinol, solidaires à leurs bases d'un tube de pincement 6, lui-même solidaire de l'extrémité distale du fil de manoeuvre 3.

Sous l'action dudit fil de manoeuvre 3, les deux boucles de fil 4 et 5 peuvent prendre :
- soit une position de capture déployée (représentée sur la figure 1), pour laquelle les deux boucles de fil 4 et 5 sont déployées en formant, à l'extérieur de l'extrémité distale de la gaine souple 2, une cage ajourée 7 au moins approximativement en forme de globe, dont lesdites boucles de fil 4 et 5 forment deux plans méridiens orthogonaux ;
- soit une position d'extraction repliée (non représentée), pour laquelle les boucles 4 et 5 sont aplaties et rétractées au moins partiellement à l'intérieur de la partie distale de la gaine souple 2.

Comme cela est illustré sur les figures 1 à 3, à leurs extrémités distales, les boucles de fil 4 et 5 se croisent en 8 en étant liées en coulissement l'une à l'autre de façon que chaque boucle de fil 4 ou 5 puisse coulisser de façon limitée par rapport à l'autre boucle de fil 5 ou 4, tout en gardant au moins approximativement son équerrage par rapport à cette dernière. Pour ce faire, comme représenté sur les figures 2 et 3, à l'emplacement du croisement 8, la boucle de fil 4 comporte un passage 9 dans lequel passe avec jeu la boucle de fil 5.

Dans les exemples de réalisation des figures 2 et 3, ledit passage 9 est formé entre le fil de ladite boucle 4 et une languette 10, découpée dans ledit fil de cette boucle 4 et conformée de façon adéquate. Sur la figure 2, la languette 10 se trouve en position intermédiaire dans la largeur du fil de la boucle 4 et est délimitée par deux lignes de coupe longitudinales 11 et 12. En revanche, sur la figure 3, la languette 10 se trouve au voisinage d'une périphérie du fil de la boucle 4 et peut donc être délimitée par une seule ligne de coupe longitudinale 13.

Bien que les exemples de réalisation décrits ci-dessus ne comportent que deux boucles de fil 4 et 5 orthogonales, on comprendra aisément que l'extracteur chirurgical conforme à la présente invention peut comporter au moins une autre boucle de fil supplémentaire (non représentée) qui, de façon analogue à la boucle 5, passerait dans le passage 9 de la boucle 4, bien entendu prévue pour que puissent y coulisser avec jeu (sous des angles différents de 90°) ladite boucle 5 et ladite boucle supplémentaire.

De même, bien que la section des fils des boucles 4 et 5 soit plate sur les figures 1 à 3, il va de soi que cette forme n'est pas nécessaire et que lesdits fils pourraient présenter une section ronde.

## Revendications

1. Extracteur chirurgical (1) pour l'extraction de corps étrangers à travers des voies naturelles ou chirurgicales, comportant :
- une gaine souple (2) apte à pénétrer dans lesdites voies jusqu'à un tel corps à extraire;
- un fil de manoeuvre (3) longitudinalement rigide, apte à coulisser dans ladite gaine souple (2) et à être manoeuvré en coulissement, depuis l'extérieur, par son extrémité proximale ; et
- deux boucles de fil (4,5) disposées à l'extrémité distale dudit fil de manoeuvre (3) et pouvant prendre, sous l'action de ce dernier :
soit une position de capture, pour laquelle lesdites boucles (4, 5) sont déployées en formant, à l'extérieur de l'extrémité distale de ladite gaine souple (2), une cage ajourée (7) en forme au moins approximative de globe, dont chaque boucle forme un plan méridien,
soit une position d'extraction, pour laquelle lesdites boucles (4, 5) sont aplaties et
rétractées au moins partiellement à l'intérieur de la partie distale de ladite gaine souple (2),
lesdites boucles de fil (4, 5) se croisant (en 8) à leurs extrémités distales en y étant liées les unes aux autres,
**caractérisé en ce que,** à son extrémité distale, le fil d'une (4) desdites boucles de fil comporte un passage (9) dans lequel passe avec jeu l'autre boucle de fil (4, 5), de sorte que, à l'emplacement du croisement distal (8) lesdites boucles de fil (4, 5) sont orthogonales, et celles-ci sont liées en coulissement, chaque boucle pouvant coulisser de façon limitée par rapport à au moins une autre boucle tout en gardant au moins approximativement, en position de capture, son équerrage par rapport à ladite autre boucle.

2. Extracteur chirurgical (1) pour l'extraction de corps étrangers à travers des voies naturelles ou chirurgicales, comportant :
- une gaine souple (2) apte à pénétrer dans lesdites voies jusqu'à un tel corps à extraire;
- un fil de manoeuvre (3) longitudinalement rigide, apte à coulisser dans ladite gaine souple (2) et à être manoeuvré en coulissement, depuis l'extérieur, par son extrémité proximale ; et
- une pluralité de boucles de fil (4,5) disposées à l'extrémité distale dudit fil de manoeuvre (3) et pouvant prendre, sous l'action de ce dernier :
soit une position de capture, pour laquelle lesdites boucles (4, 5) sont déployées en formant, à l'extérieur de l'extrémité distale de ladite gaine souple (2), une cage ajourée (7) en forme au moins approximative de globe, dont chaque boucle forme un plan méridien,
soit une position d'extraction, pour laquelle lesdites boucles (4, 5) sont aplaties et
rétractées au moins partiellement à l'intérieur de la partie distale de ladite gaine souple (2),
lesdites boucles de fil (4, 5) se croisant (en 8) à leurs extrémités distales en y étant liées les unes aux autres,
**caractérisé en ce que,** à son extrémité distale, le fil d'une (4) desdites boucles de fil comporte un passage (9) dans lequel passe avec jeu une autre boucle de fil (4, 5), de sorte que, à l'emplacement du croisement distal (8) lesdites boucles de fil (4, 5) sont orthogonales, et celles-ci sont liées en coulissement, chaque boucle pouvant coulisser de façon limitée par rapport à au moins une autre boucle tout en gardant au moins approximativement, en position de capture, son équerrage par rapport à ladite autre boucle, et **caractérise en outre en ce que** si il y a au moins une autre boucle de fil supplémentaire, elle passerait de façon analogue à la deuxième boucle (4,5) dans le passage (9) du fil de la première boucle prévue pour qu'elle puisse y coulisser avec jeu sous des angles différents de 90°.

3. Extracteur chirurgical selon la revendication 1 ou la revendication 2, **caractérisé en ce que** ledit passage (9) est formé par une languette (10) réalisée dans le fil de la boucle de fil correspondante (4).

## Claims

1. Surgical extractor (1) for extracting foreign bodies via natural or surgical routes, comprising:
- a flexible sheath (2) capable of entering said routes up to such a body to be extracted;
- a longitudinally rigid manoeuvring wire (3), capable of sliding in said flexible sheath (2) and being manoeuvred in a sliding fashion, from the outside, via the proximal end thereof; and
- two wire loops (4, 5) arranged at the distal end of said manoeuvring wire (3) and capable of adopting, under the action thereof:
either a capture position, for which said loops (4, 5) are deployed by forming, outside the distal end of said flexible sheath (2), an open-worked cage (7) having at least approximately the shape of a globe, wherein each loop forms a meridian plane,
or an extraction position, for which said loops (4, 5) are flattened and retracted at least partially inside the distal part of said flexible sheath (2),
said wire loops (4, 5) intersecting (at 8) at the distal ends thereof while being attached to each other,
**characterised in that,** at the distal end thereof, the wire of one (4) of said wire loops comprises a passage (9) wherein the other wire loop (4, 5) fits with play, such that, at the position of the distal intersection (8) of said wire loops (4, 5) are orthogonal, and said loops are attached in a sliding manner, each loop being capable of sliding in a limited manner with respect to at least one other loop while retaining at least approximately, in the capture position, the squareness thereof with respect to said other loop.

2. Surgical extractor (1) for extracting foreign bodies via natural or surgical routes, comprising:
- a flexible sheath (2) capable of entering said routes up to such a body to be extracted;
- a longitudinally rigid manoeuvring wire (3), capable of sliding in said flexible sheath (2) and being manoeuvred in a sliding fashion, from the outside, via the proximal end thereof; and
- a plurality of wire loops (4, 5) arranged at the distal end of said manoeuvring wire (3) and capable of adopting, under the action thereof:
either a capture position, for which said loops (4, 5) are deployed by forming, outside the distal end of said flexible sheath (2), an open-worked cage (7) having at least approximately the shape of a globe, wherein each loop forms a meridian plane,
or an extraction position, for which said loops (4, 5) are flattened and retracted at least partially inside the distal part of said flexible sheath (2),
said wire loops (4, 5) intersecting (at 8) at the distal ends thereof while being attached to each other,
**characterised in that,** at the distal end thereof, the wire of one (4) of said wire loops comprises a passage (9) wherein the other wire loop (4, 5) fits with play, such that, at the position of the distal intersection (8) of said wire loops (4, 5) are orthogonal, and said loops are attached in a sliding manner, each loop being capable of sliding in a limited manner with respect to at least one other loop while retaining at least approximately, in the capture position, the squareness thereof with respect to said other loop, and also **characterised in that**, if there is at least one other additional wire loop, it would fit in a similar fashion to the second loop (4, 5) in the passage (9) of the wire of the first loop provided so that it can slide with play at angles different to 90°.

3. Surgical extractor according to claim 1 or claim 2, **characterised in that** said passage (9) is formed by a tab (10) produced in the wire of the corresponding wire loop (4).

## Patentansprüche

1. Chirurgischer Extraktor (1) zur Extraktion von Fremdkörpern über natürliche oder chirurgische Zugänge, der Folgendes umfasst:
- eine biegsame Hülle (2), die dazu geeignet ist, in die besagten Zugänge bis zu einem solchen zu extrahierenden Körper einzudringen;
- einen längs verlaufenden starren Manövrierdraht (3), der dazu geeignet ist, in der besagten biegsamen Hülle (2) zu gleiten und gleitend von der Außenseite aus über sein proximales Ende manövriert zu werden; und
- zwei Drahtösen (4, 5), die am distalen Ende des besagten Manövrierdrahts (3) angeordnet sind und die mittels Betätigung des letzteren Folgendes einnehmen können:
eine Erfassungsposition, in der die besagten Ösen (4, 5) aufgeweitet sind, um an der Außenseite des distalen Endes der besagten biegsamen Hülle (2) einen Drahtkäfig (7) in einer zumindest ungefähren Kugelform zu bilden, in dem jede Öse eine Meridianebene bildet,
eine Extraktionsposition, in der die besagten Ösen (4, 5) abgeflacht und zumindest zum Teil in das Innere des distalen Teils der besagten biegsamen Hülle (2) zurückgezogen sind,
wobei die besagten Drahtösen (4, 5) sich an ihren distalen Enden kreuzen (bei 8), um so miteinander verbunden zu sein,
**dadurch gekennzeichnet, dass** der Draht einer (4) der besagten Drahtösen an seinem distalen Ende einen Durchlass (9) umfasst, durch den die andere Drahtöse (4, 5) mit Spiel verläuft, so dass die besagten Drahtösen (4, 5) an der Stelle der distalen Kreuzung (8) orthogonal sind und diese gleitend verbunden sind, wobei jede Öse auf eingeschränkte Weise in Bezug auf mindestens eine andere Öse gleiten kann, indem ihre Ausrichtung in Bezug auf die besagte andere Öse in der Erfassungsposition zumindest ungefähr beibehalten wird.

2. Chirurgischer Extraktor (1) zur Extraktion von Fremdkörpern über natürliche oder chirurgische Zugänge, der Folgendes umfasst:
- eine biegsame Hülle (2), die dazu geeignet ist, in die besagten Zugänge bis zu einem solchen zu extrahierenden Körper einzudringen;
- einen längs verlaufenden starren Manövrierdraht (3), der dazu geeignet ist, in der besagten biegsamen Hülle (2) zu gleiten und gleitend von der Außenseite aus über sein proximales Ende manövriert zu werden; und
- mehrere Drahtösen (4, 5), die am distalen Ende des besagten Manövrierdrahts (3) angeordnet sind und die mittels Betätigung des letzteren Folgendes einnehmen können:
eine Erfassungsposition, in der die besagten Ösen (4, 5) aufgeweitet sind, um an der Außenseite des distalen Endes der besagten biegsamen Hülle (2) einen Drahtkäfig (7) in einer zumindest ungefähren Kugelform zu bilden, in dem jede Öse eine Meridianebene bildet,
eine Extraktionsposition, in der die besagten Ösen (4, 5) abgeflacht und zumindest zum Teil in das Innere des distalen Teils der besagten biegsamen Hülle (2) zurückgezogen sind,
wobei die besagten Drahtösen (4, 5) sich an ihren distalen Enden kreuzen (bei 8), um so miteinander verbunden zu sein,
**dadurch gekennzeichnet, dass** der Draht einer (4) der besagten Drahtösen an seinem distalen Ende einen Durchlass (9) umfasst, durch den eine andere Drahtöse (4, 5) mit Spiel verläuft, so dass die besagten Drahtösen (4, 5) an der Stelle der distalen Kreuzung (8) orthogonal sind und diese gleitend verbunden sind, wobei jede Öse auf eingeschränkte Weise in Bezug auf mindestens eine andere Öse gleiten kann, indem ihre Ausrichtung in Bezug auf die besagte andere Öse in der Erfassungsposition zumindest ungefähr beibehalten wird, und außerdem **dadurch gekennzeichnet, dass**, wenn er mindestens eine andere zusätzliche Drahtöse aufweist, sie auf ähnliche Weise zu der zweiten Öse (4,5) durch den Durchlass (9) des Drahts der ersten Öse verläuft, die dazu vorgesehen ist, dass sie mit Spiel in unterschiedlichen Winkeln von 90° gleiten kann.

3. Chirurgischer Extraktor nach Anspruch 1, oder 2, **dadurch gekennzeichnet, dass** der besagte Durchlass (9) von einer Lasche (10) gebildet wird, die in dem Draht der entsprechenden Drahtöse (4) hergestellt wurde.
